# EUROPEAN PATENT SPECIFICATION

(11) **EP 4 087 051 B1**
(45) Date of publication and mention of the grant of the patent: **11.06.2025**
(21) Application number: 21171782.2
(22) Date of filing: 03.05.2021
(51) Int. Cl.: H01P 1/06, A61B 6/00, A61B 6/03

(54) **ROTARY TRANSMISSION SYSTEM USING A WAVEGUIDE**
DREHÜBERTRAGUNGSSYSTEM MIT VERWENDUNG EINES WELLENLEITERS
SYSTÈME DE TRANSMISSION ROTATIVE À L'AIDE D'UN GUIDE D'ONDES

(43) Date of publication of application: 09.11.2022
(73) Proprietor: Schleifring GmbH, 82256 Fürstenfeldbruck (DE)
(72) Inventor: PANHANS, Christian, 86150 Augsburg (DE); STOLLE, Reinhard, 86561 Aresing (DE)
(74) Representative: Lohr, Jöstingmeier & Partner

(56) References cited:
- US-A- 2 945 193
- US-A1- 2020 194 861
- "Electromagnetic Waves and Antennas", vol. 10111113, 1 August 2016, article ORFANIDIS SOPHOCLES J.: "Ch. 9: Waveguides", pages: 362 - 410, XP055847229

## Description

### Field of the invention

The invention relates to a rotary transmission system for providing a non-contact high speed datalink between rotating devices and which may specifically be used for computed tomography scanners.

### Description of the related art

Non-contact data links may be used to couple rotating devices like the rotating part of the gantry of a computed tomography (CT) scanner to the stationary part. Data rates are in the above 1Gbit/s or even more than 10 Gbit/s range. Such data links may also be called a rotary joint or a slipring.

As CT scanners have a large inner bore for accommodation of a patient to be scanned, rotary joints used therein must have a large diameter, which normally is in the 1 - 1.5m range. US 5,646,962 A discloses such a non-contact rotary joint which is based on a stripline for guiding a signal around a circular body and a capacitive pickup movable thereto for receiving said signal.

Another approach as disclosed in EP 0 093 468 A1 uses a waveguide for transmitting signals. A stationary waveguide, comprising a circular shaped, conductive hollow body has a fixed receiving antenna. Further, a rotatable transmission antenna is movable in a radial slot in the body to couple signals into the interior of the waveguide. These signals travel along the waveguide until they reach the receiving antenna.

US 2020/194861 A1 and US 2 945 193 A disclose rotary waveguide joints for transmitting microwave signals.

A problem is the comparatively small relative bandwidth of the waveguide, which limits the usable data rate. Further, the radial slot in the waveguide must be comparatively narrow to avoid degradation of the waveguide. The transmission antenna must fit into this slot and therefore can only be a small pin. Such an antenna is limited in its bandwidth and efficiency.

### Summary of the invention

The problem to be solved by the invention is to provide improved high-speed coupling between rotatable parts.

Solutions of the problem are described in the independent claims. The dependent claims relate to further improvements of the invention.

A rotary joint which includes a high speed data link between rotatable parts comprises a circular channel. The circular channel may include an inner ring and an outer ring. Both rings are rotatable relative to each other around an axis which may be the rotation axis of the rotary joint. The outer ring has a larger diameter than the inner ring, thereby providing a radial gap or a space between the rings. Further, the inner ring and the outer ring may be aligned axially. Basically, any of the rings may be stationary, whereas the other is rotatable.

The circular channel further may include at least one or two sidewalls. A first sidewall is located axially on one side of the rings, whereas the second sidewall is located axially on the other or opposing side of the rings. The inner ring, the outer ring, the first sidewall and the second sidewall form a circular channel which may have a rectangular or square cross-section. Basically, any of the sidewalls may be stationary, or rotatable.

The sidewalls are not necessary for the transmission function of the channel but can help to decouple the signal transmitted from the environment or neighboring channels by using reflective or absorptive sidewalls. Thus, also a standard wireless bus as e.g. IEEE 802.11 can be used without interfering with external wireless bus installations used for other purposes.

For transmitting signals, an inner antenna is mechanically coupled to the inner ring and an outer antenna is mechanically coupled to the outer ring. The inner antenna and the outer antenna are directed into a space between the inner ring and the outer ring. This allows to transfer microwave signals between the inner antenna and the outer antenna, if one of the antennas radiates signals into the channel and the other antenna receives signals from the channel. The channel and specifically the inner ring and the outer ring may reflect microwave signals, such that the microwave signals propagate through the ring. To improve reflection of the signals, the channel and specifically the rings and/or the sidewalls may include an electrically conductive material, which may be a metal. They may be made of such a conductive material, or they may have a surface coated with such a conductive material.

The rings (210, 220) and/or sidewalls (230, 240) may be of an electromagnetically reflective material e.g. conductive material or have a conductive surface or are of a dielectric material with high permittivity.

Such a rotary joint may be used in a gantry of a CT scanner which includes a stationary part and a rotating disk, which rotates about a rotation axis. The rotating disk may hold components like a power supply, an X-ray tube, an X-ray detector, and a data acquisition system. The rotary joint may receive data from the data acquisition system and couple these to the stationary part.

The channel may have a rectangular or square cross-section. Therefore, it may be described as a hollow rectangular or square toroid. Normally, the channel may be a void space, filled with air, but it may also include a dielectric material, which may at least partially fill the channel.

In an embodiment, the inner ring may be mounted and/or connected to the first sidewall, and the outer ring may be mounted and/or connected to the second sidewall, thus for. This results in two parts which are rotatable against each other. There may be two gaps between these two parts which may be very close or which may be bridged by sliding contacts, springs, a gasket or any other means which may provide low electrical impedance between these two parts, which may be a capacitive overlap. This improves shielding and avoids unwanted radiation. It is basically the same, if the inner ring is mounted and/or connected to the second sidewall and the outer ring is mounted and/or connected to the first sidewall.

In an embodiment, the inner ring may be mounted to the first sidewall and to the second sidewall, such that the inner ring is rotatable together with the first and second sidewalls. To allow a rotation, a gap may be provided on both sides of the outer ring, such that the outer ring can move freely against the inner ring and the sidewalls. An inverted embodiment may include an outer ring mounted to the first sidewall and the second sidewall.

Basically, there may be any combination of parts connected, as long as the inner ring is rotatable against the outer ring. Any gap between rotatable parts may be bridged by sliding contacts, springs, a gasket or any other means which may provide low electrical impedance between these two parts, which may be a capacitive overlap. The gap may have a width of 0,5 to 4mm, which may be just big enough to allow rotation and mechanical tolerances of objects of that size.

In an embodiment, the inner ring and the outer ring may have the same width and are axially aligned. Further, the first sidewall and the second sidewall may be flat disk-shaped rings covering the space between the inner ring and the outer ring. At least one of the sidewalls may overlap at least one the rings to bridge a gap there-between and to provide at least a capacitive coupling. The overlap might be dimensioned to be a quarter or multiple quarters of the wavelength.

The distance between the inner ring and the outer ring is close to five times the wavelength. According to an embodiment defined by the claims, the distance is five times the wavelength with a tolerance of plus or minus 50%. This may allow for lowest dispersion between the signal paths with lowest and highest number of reflections.

In an embodiment, the inner antenna and/or the outer antenna have an adjustable directivity wherein transmitted power and receiver sensitivity together with conductivity of the reflective surface of inner and outer ring may be configured for a predetermined number of reflections between the rings leading to a sufficiently low signal after one round of propagation.

In an embodiment, the inner antenna and/or the outer antenna are directed into the circular channel. If a microwave signal is radiated into the channel, it will be reflected by the channel, such that it can be received from the channel. Therefore, in general the embodiments may work with unspecific antennas simply radiating into the channel and receiving from the channel. Improved transmission may be achieved by directing the inner antenna towards the outer ring and the outer antenna towards the inner ring. This ensures proper reflection by the rings to transfer the signals. In a further embodiment, the antennas may have a configurable directivity. This means that they have an adjustable radiation pattern. This may simply be achieved by phased array antennas. The radiation pattern may be adjusted such that a specific angle of reflection is obtained which may result in a well-defined signal path between the inner antenna and the outer antenna. There may also be different or multiple signal paths which may further be used for multiple channels. This may increase transmission rate and/or transmission quality. In an example, there may be a first signal path having two reflections, and another signal path having three reflections. These may be well separated due to their different reflection angles by selective antennas. There may be any number of signal paths.

Another embodiment, which does not form part of the claimed invention, relates to a data link between movable parts comprising a hollow channel in general. This hollow channel may have a linear shape, but it may also have any other shape like a combination of linear and/or curved sections. The hollow channel may include four sidewalls which may define a rectangular or squared cross-section. This embodiment is comparable to the circular channel embodiments disclosed herein. The linear hollow channel may include a first sidewall with a first antenna, and a second sidewall opposing thereto with a second antenna. The first and second sidewalls may be parallel to each other to allow reflections between the first and second sidewalls. There may be third and fourth sidewalls at the sides of the first and second sidewalls to form the hollow channel.

The circular channel may have a height and a width. Also, the linear hollow channel may have a height and a width. The channel can only guide microwave signals if a wavelength of the microwave signal is shorter than 2 times the width or the height, whichever is larger. Embodiments which only partially fall within the scope of the claims work best if at least one wavelength of the microwave signals, which may have multiple different wavelengths, is shorter than ½, 1/3, ¼, 1/5, or 1/10, or 1/20 or 1/50, or 1/100 of the width or the height, whichever is larger. The smaller the wavelength of the signal is compared to the width or to the height of the channel, the more transmission paths under different angles are possible. As the main transmission may be reflection between the inner and the outer ring, or between the first and second sidewalls, at least one wavelength of the microwave signal may be shorter than 1/2 , 1/3, ¼, 1/5, or 1/10, or 1/20 or 1/50, or 1/100 of this distance.

The distance between the inner ring and the outer ring may be significantly larger than half of a wavelength of the transmitted signal to allow for a multimode propagation of the signal.

Herein, the term microwave is used for radiofrequency signals which are in a range of more than 300 MHz. In an embodiment, signals in a range above 2 GHz may be used. Very good transmission characteristics have been obtained with signals in a 60 GHz range. The embodiments may also use frequencies of several 100 GHz or more.

In an embodiment, the data transmission implemented may conform to a wireless standard e.g. IEEE802.11 ad or ay.

The dimensions of inner and outer ring may be optimized to achieve a typical number of reflections when the microwave signal is propagating one round. The strategy to optimize the angle is to find an angle where the antenna has a defined directivity, resulting in a transmitted narrow beam of high amplitude with a strong attenuation outside the beam and few side lobes. The same directivity profile may be present at the receive side. Also, the angle may not be as steep, such that the reflections at the opposing ring go back into the antenna. This may be achieved with a two-dimensional patch antenna as phased array antenna with sufficient angular resolution.

Also, the reflection attenuation defined by conductivity and angle of reflection may be optimized, a higher conductivity of the material leads to a lower attenuation, a lower conductivity to a higher attenuation. The strategy is to reduce dispersion (delay spread) between signals having a different number of reflections because of the higher attenuation caused with every reflection.

One angle of reflection is preferred by the directivity characteristic of the main beam of the antenna and side lobes may be present but experience a higher reflection attenuation that additionally attenuates these paths so that the majority of the signal paths varies only slightly in regard to its total path lengths and angles.

Axial displacement of transmitter and receiver may reduce the dynamic range of received signal strengths between minimum signal path 0 degrees and signal path 360 degrees.

Attenuating material may be mounted to at least one of the rings axially to the antenna and in the vicinity of the antenna to attenuate a part of the signal that propagates more than a full round, reducing the interference of directly received signal and signal that propagates more than one round.

An embodiment may use a feature of the wireless standards as e.g. IEEE802.11 ad or ay: the guard interval together with OFDM or single carrier with frequency domain equalization. The transceiver may periodically train on the characteristics of the channel, multiple transmission paths like clockwise and counterclockwise transmission can be employed. A guard interval of the standard applied may be selected such that it is shorter than the signal propagation time through the channel. For a given guard interval, the distance between the inner ring and the outer ring may be adapted to obtain a predetermined maximum path length resulting in a predetermined maximum signal propagation time.

Guard intervals of the above-mentioned wireless standards may be used to allow for multi-path propagation. There may be a training to optimize the guard intervals.

There might be several parallel channels arranged radially or axially. The axial arrangement is preferred with multiple antennas coupled having sidewalls to separate channels. Thus, the total transmission capacity can be increased when there is sufficient attenuation between the channels.

The transmit and receive frequencies might be different at a transceiver to have a better signal separation between the communication channels.

### Description of Drawings

In the following the invention will be described by way of example, without limitation of the general inventive concept, on examples of embodiment with reference to the drawings.
Figure 1 shows an embodiment.
Figure 2 shows a sectional side view of the circular channel.
Figure 3 shows a front view into the circular channel.
Figure 4 shows a further front view into the circular channel.
Figure 5 shows a dual path propagation.
Figure 6 shows a multipath propagation.
Figure 7 shows a linear embodiment.
Figure 8 shows an exemplary functional block diagram.

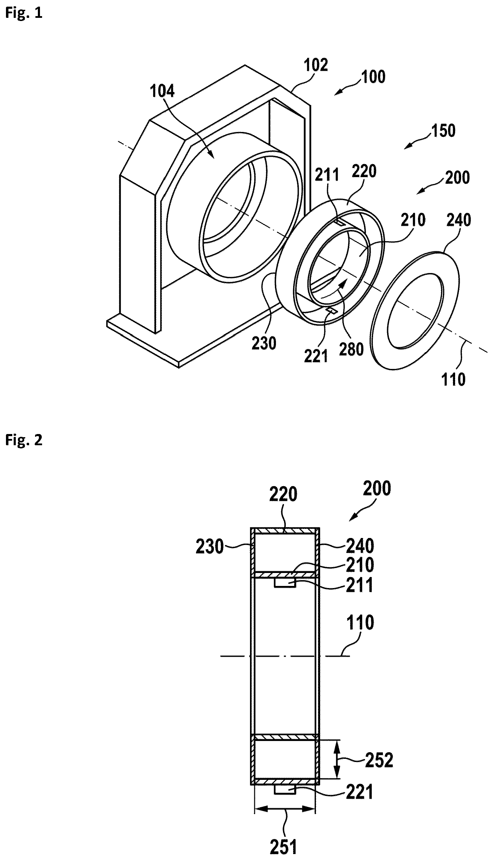

In figure 1 a first embodiment is shown. A gantry 100 of a CT scanner comprises a stationary part 102 and a rotating disk 104, rotating about a rotation axis 110. The rotating disk may hold rotating components which are not shown here, like a power supply, an X-ray tube, an X-ray detector and a data acquisition system. Further, a slip ring or a rotating power transformer which is also not shown, may be provided for transfer of power from the stationary to the rotating part.

A rotary joint 150 for high speed data transmission includes a circular shaped channel 200. It may be described as a hollow rectangular or square toroid. The channel 200 encloses a hollow-cylindric volume. The channel 200 may include an inner ring 210 and an outer ring 220, both rings may be on the same axis. The embodiment would also work with offset axes. Both rings are rotatable against each other. Any one of the rings may be stationary, whereas the other may be rotatable.

To form a channel 200, a first sidewall 230 and/or a second sidewall 240 may be provided. Also, at least one of the sidewalls may be part of a gantry 100 of a CT scanner. Each sidewall may be fixed to one of the rings 210, 220. There may also be a low impedance contact between a sidewall and a ring. To the other ring there may be a gap, which may be bridged by sliding brushes, a conductive gasket or any other suitable material which may provide a good electrical contact.

In an embodiment, the ring 210 and both sidewalls 230, 240 may be connected together forming an U-shaped cross section, while ring 220 is rotatable. There may be gaps at the sides of the outer ring 220 to allow for rotation. There may be any other combination of connected parts which may allow rotation of the rings 210 and 220 with their mechanically coupled antennas 211 and 221 and may form a toroid with rectangular section together with sidewalls.

Both rings 210, 220 may have the same length and may be axially aligned. The sidewalls 230, 240 may be flat disk shaped rings, they may also overlap at least one of the inner ring 210 and the outer ring 220.

The rings 210, 220 and the sidewalls 239, 240 comprise an electrically conductive material like a metal and or a material with electrically conductive surfaces.

An inner antenna 211 is at the inner ring 210 while an outer antenna 221 is at the outer ring 220. The antennas may rotate or be stationary with their rings, the antennas being mounted to the rings.

Figure 2 shows a sectional side view of the circular channel 200. The channel may have a rectangular or squared cross section with a width 251 between the sidewalls 230, 240 and a height 252 between the rings 210, 220.

The waveguide has an inner space which allows the propagation of electromagnetic waves with a maximum wavelength λk equals to two times the width 251 or the height 252, whichever is larger.

Since the sidewalls 230, 240 are not necessary for the function they can be omitted, then the width of the channel may be the smallest width of the rings 210, 220.

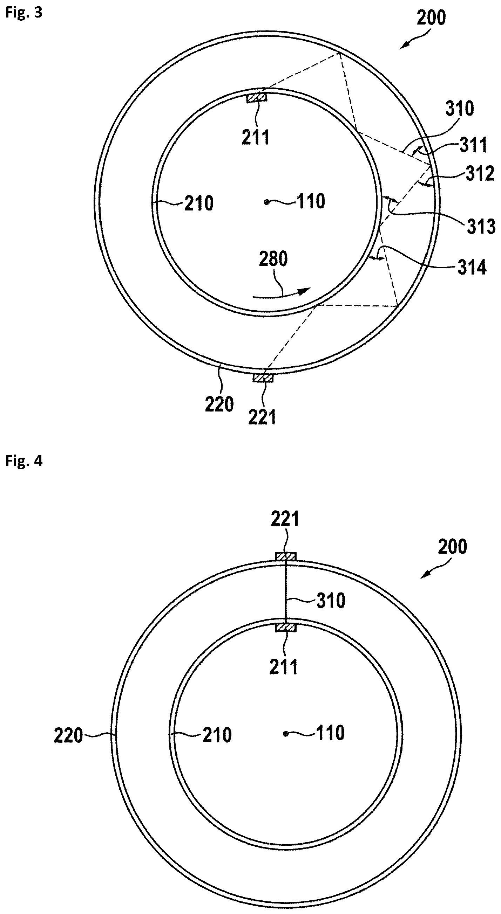

Figure 3 shows a front view into the circular channel 200 with a possible signal path between inner antenna 211 and outer antenna 221. The signal may not only be transmitted in a single mode within the circular channel 200, but it may also be reflected at the inner ring 210 and or the outer ring 220. In this figure, inner antenna 211 and outer antenna 221 have a relative angle of about 180 degrees. There may be multiple reflections at the rings as shown and dependent of the specific direction of radiation of the antennas. With each reflection the angles of the electromagnetic wave 310 to be reflected and the reflected wave versus a surface of a ring are the same. Such, the first angle at outer ring 311 is the same as the second angle at outer ring 312 and the first angle at inner ring 313 is the same as second angle at inner ring 314.

Figure 4 shows a further front view into the circular channel 200. In this figure, inner antenna 211 and outer antenna 221 have a relative angle of about 0 degrees, such that they are opposing each other. Here, the electromagnetic wave 310 may directly propagate from inner antenna 211 to outer antenna 221.

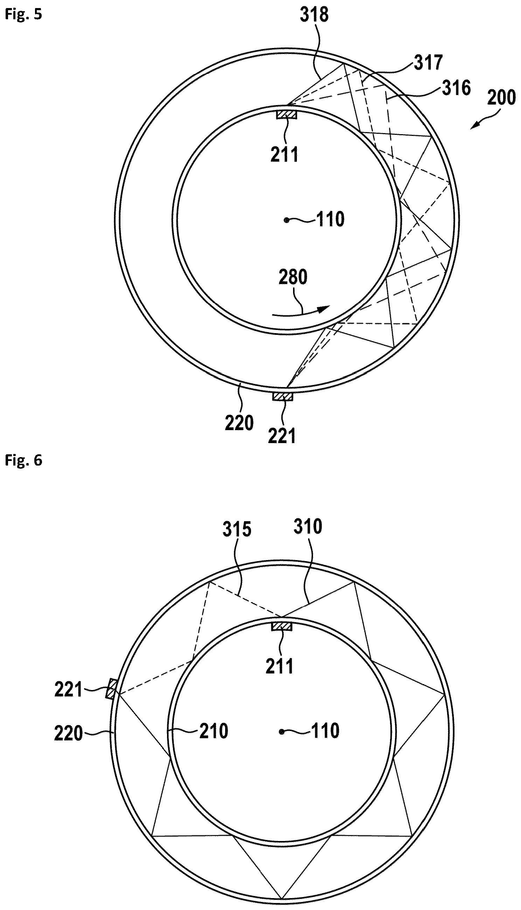

Figure 5 shows a dual path propagation. In this figure, inner antenna 211 and outer antenna 221 have a relative angle of about 270 degrees. Here, the electromagnetic wave 310 may propagate clockwise from inner antenna 211 to outer antenna 221. There may also be a second counterclockwise signal path 315, if the antennas are set to radiate accordingly. The antennas and/or transmitter and/or receiver may switch between these signal paths to select the best one. Both signal paths may also be used to increase date rate. Further, both signal paths may be used for bidirectional signal transmission. This example shows a relative angle of about 270 degrees, but two signal paths are basically available through all relative angles between inner antenna 211 and outer antenna 221.

Figure 6 shows a multipath propagation. In this figure, inner antenna 211 and outer antenna 221 have a relative angle of about 180 degrees. Here three different multipath propagations 316, 317, 318 are shown. This may be used to further increase bandwidth.

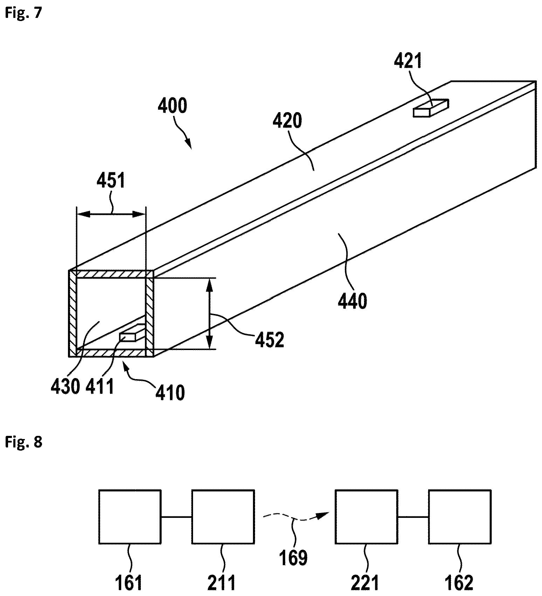

Figure 7 shows a linear embodiment. A hollow channel 400 includes four sidewalls 410, 420, 430, 440, defining a rectangular cross sectioned or square cross sectioned hollow space. A first sidewall 410 is parallel to a second sidewall 420. Further, a first antenna 411 is mechanically coupled to the first sidewall 410 and a second antenna 421 is movable within the linear channel. The second antenna may be coupled to the second sidewall. The sidewalls 410, 420, 430, 440 include an electrically conductive material like a metal. They may be made from such a material or they may have a conductive surface which may include such a material. The first antenna 411 and the second antenna 421 are configured for a microwave signal connection 169 between them. This embodiment is basically the same as the circular embodiments disclosed herein, but is linear. The first sidewall 410 corresponds to the inner ring 210 and the second sidewall 420 corresponds to the outer ring 220. Further, an embodiment may have any shape like a combination of curved sections and/or linear sections.

Figure 8 shows an exemplary functional block diagram. A transmitter 161, which may be fed by a data acquisition system providing imaging data sends signals to inner antenna 211 which radiates microwave signals 169 into the circular channel 200. These RF signals 169 are received by antenna 221 and forwarded to a receiver 162. Basically, the direction may be reversed. Also a bidirectional communication may be made.

### List of reference numerals

- 100: gantry of CT scanner
- 102: stationary part
- 104: rotatable disk
- 110: rotation axis
- 150: rotary joint
- 161: transmitter
- 162: receiver
- 169: RF signals
- 200: circular channel
- 210: inner ring
- 211: inner antenna
- 220: outer ring
- 221: outer antenna
- 230: first sidewall
- 240: second sidewall
- 251: width
- 252: height
- 310: electromagnetic wave propagation
- 311: first angle at outer ring
- 312: second angle at outer ring
- 313: first angle at inner ring
- 314: second angle at inner ring
- 315: alternate electromagnetic wave propagation
- 316: first multipath propagation
- 317: second multipath propagation
- 318: third multipath propagation
- 400: hollow channel
- 410: first sidewall
- 420: second sidewall
- 411: first antenna
- 420: second sidewall
- 421: second antenna
- 430: third sidewall
- 440: fourth sidewall
- 451: width
- 452: height

## Claims

1. A rotary joint providing a high speed datalink between rotatable parts comprising a circular channel (200),
the circular channel (200) comprises:
- an inner ring (210) and an outer ring (220),
both rings being centered about a common rotation axis (110) and being rotatable against each other, and
the outer ring (220) having a larger diameter than the inner ring (210),
- an inner antenna (211) mechanically coupled to the inner ring (210), and
- an outer antenna (221) mechanically coupled to the outer ring (220),
wherein
the inner antenna (211) and the outer antenna (221) are directed into a space between the inner ring (210) and the outer ring (220) and configured for a microwave signal connection (169) between them,
**characterized in that**
the distance between the inner ring and the outer ring is 5 times the wavelength of the microwave signal with a tolerance of plus or minus 50%.

2. A rotary joint according to claim 1,
wherein a first sidewall (230) axially on one side of the rings and a second sidewall (240) opposing the first sidewall (230) axially on the other side of the rings are provided.

3. A rotary joint according to claim 2,
wherein the inner ring (210) is mounted to the first sidewall (230) and the outer ring (220) is mounted to the second sidewall (240) and
a first gap is formed between the inner ring (210) and the second sidewall (240) and
a second gap is formed between the outer ring (220) and the first sidewall (230).

4. A rotary joint according to claim 2,
wherein one of the inner ring (210) or outer ring (220) is mounted to the first sidewall (230) and the second sidewall (240), forming a gap on each side of the other one of outer ring (220) or inner ring (210) to the first sidewall (230) and the second sidewall (240).

5. A rotary joint according to claim 3 or 4,
wherein at least one of the gaps is bridged by at least a sliding contact, a gasket, a seal or an overlap providing capacitive coupling.

6. A rotary joint according to any of claims 2 to 5,
wherein the first sidewall (230) and/or the second sidewall (240) is disc shaped and has a center hole.

7. A rotary joint according to any of the claims 2 to 6,
wherein at least one of the rings (210, 220) is of an electromagnetically reflective material e.g. conductive material or has a conductive surface or at least one of the rings (210, 220) and/or sidewalls (230, 240) are of a dielectric material with high permittivity.

8. A rotary joint according to any of the claims 2 to 7,
wherein at least one of the first sidewall (230) and second sidewall (240) includes an absorbing and/or reflecting material for the microwave signal or includes a combination of an absorbing material mounted to a conductive surface.

9. A rotary joint according to any of the claims 2 to 8,
wherein the circular channel (200) defines a width (251) between the sidewalls (230, 240) and a height (252) between the rings (210, 220.

10. A rotary joint according to any of the previous claims,
wherein the circular channel (200) encloses a hollow-cylindric volume.

11. A rotary joint according to claim 10,
wherein either the inner antenna (211) and/or the outer antenna (221) have an adjustable directivity
or the inner antenna (211) and/or the outer antenna (221) have an adjustable directivity wherein at least the angle of radiation is configured for a predetermined value or a position dependent value.

12. A rotary joint according to any of claims 10 or 11,
wherein the antenna directivity is optimized to a beam which has its first reflection at the opposing ring and its second reflection outside the antenna.

13. A rotary joint according to any of claims 10 to 12,
wherein the inner antenna (211) and/or the outer antenna (221) have an adjustable directivity wherein transmitted power and receiver sensitivity together with conductivity of the reflective surface of inner and outer ring are configured for a predetermined number of reflections between the rings (210,220).

14. A rotary joint according to any of the previous claims,
comprising at least one of the following features:
a. the inner antenna (211) and the outer antenna (221) are axially displaced to each other,
b. the inner ring (210) has attenuating material fixed axially to the inner antenna,
c. the outer ring (220) has attenuating material fixed axially to the outer antenna,
d. the inner antenna (211) is directed into the channel (200),
e. the outer antenna (221) is directed into the channel (200),
f. the inner antenna (211) is directed towards the outer ring (220),
g. the outer antenna (221) is directed towards the inner ring (210).

## Patentansprüche

1. Ein Drehübertrager, bereitstellend eine Hochgeschwindigkeitsdatenverbindung zwischen rotierbaren Teilen, umfassend einen kreisförmigen Kanal (200),
wobei der kreisförmige Kanal (200) umfasst:
- einen inneren Ring (210) und einen äußeren Ring (220),
wobei beide Ringe um eine gemeinsame Rotationsachse (110) zentriert sind und gegeneinander rotierbar sind, und
- eine innere Antenne (211), welche mechanisch mit dem inneren Ring (210) gekoppelt ist, und
- eine äußere Antenne (221), welche mechanisch mit dem äußeren Ring (220) gekoppelt ist,
wobei
die innere Antenne (211) und die äußere Antenne (221) in eine Richtung zwischen dem inneren Ring (210) und dem äußeren Ring (220) gerichtet sind und für eine Mikrowellensignalverbindung (169) zwischen ihnen konfiguriert sind,
**dadurch gekennzeichnet, dass**
der Abstand zwischen dem inneren Ring und dem äußeren Ring das 5fache der Wellenlänge des Mikrowellensignals mit einer Toleranz von plus oder minus 50% beträgt.

2. Ein Drehübertrager gemäß Anspruch 1,
wobei eine erste Seitenwand (230) axial auf der einen Seite der Ringe und eine zweite Seitenwand (240) gegenüber der ersten Seitenwand (230) axial auf der anderen Seite der Ringe bereitgestellt sind.

3. Ein Drehübertrager gemäß Anspruch 2,
wobei der innere Ring (210) an der ersten Seitenwand (230) befestigt ist und der äußere Ring (220) an der zweiten Seitenwand (240) befestigt ist und
ein erster Zwischenraum zwischen dem inneren Ring (210) und der zweiten Seitenwand (240) gebildet wird und
ein zweiter Zwischenraum zwischen dem äußeren Ring (220) und der ersten Seitenwand (230) gebildet wird.

4. Ein Drehübertrager gemäß Anspruch 2,
wobei einer von dem inneren Ring (210) oder äußeren Ring (220) an die erste Seitenwand (230) und die zweite Seitenwand (240) befestigt ist, einen Zwischenraum auf jeder Seite des anderen von dem äußeren Ring (220) oder inneren Ring (210) zur ersten Seitenwand (230) und der zweiten Seitenwand (240) bildend.

5. Ein Drehübertrager gemäß Anspruch 3 oder 4,
wobei mindestens einer der Zwischenräume durch mindestens einen Schleifkontakt, eine Dichtung, eine Versiegelung oder eine Überlappung überbrückt wird, eine kapazitive Kopplung bereitstellend.

6. Ein Drehübertrager gemäß irgendeinem der Ansprüche 2 bis 5,
wobei die erste Seitenwand (230) und/oder die zweite Seitenwand (240) scheibenförmig ist und ein Mittelloch hat.

7. Ein Drehübertrager gemäß irgendeinem der Ansprüche 2 bis 6,
wobei mindestens einer der Ringe (210, 220) aus einem elektromagnetisch reflektiven Material besteht, beispielsweise einem leitfähigen Material oder eine leitfähige Oberfläche hat oder mindestens einer der Ringe (210, 220) und/oder Seitenwände (230, 240) oder aus einem dielektrischen Material mit hoher Permittivität besteht.

8. Ein Drehübertrager gemäß irgendeinem der Ansprüche 2 bis 7,
wobei mindestens eine von der ersten Seitenwand (230) und der zweiten Seitenwand (240) ein absorbierendes und/oder reflektierendes Material für das Mikrowellensignal enthält oder eine Kombination aus einem absorbierenden Material, das auf einer leitfähigen Oberfläche angebracht ist, enthält.

9. Ein Drehübertrager gemäß irgendeinem der Ansprüche 2 bis 8,
wobei der kreisförmige Kanal (200) eine Breite (251) zwischen den Seitenwänden (230, 240) und eine Höhe zwischen den Ringen (210, 220) definiert.

10. Ein Drehübertrager gemäß einem der vorhergehenden Ansprüche,
wobei der kreisförmige Kanal (200) ein hohlzylindrisches Volumen umschließt.

11. Ein Drehübertrager gemäß Anspruch 10,
wobei entweder die innere Antenne (211) und/oder die äußere Antenne (221) eine einstellbare Richtwirkung aufweisen,
oder die innere Antenne (211) und/oder die äußere Antenne (221) eine einstellbare Richtwirkung aufweisen, wobei zumindest der Abstrahlwinkel für einen vorgegebenen Wert oder einen positionsabhängigen Wert konfiguriert ist.

12. Ein Drehübertrager gemäß irgendeinem der Ansprüche 10 oder 11,
wobei die Antennenrichtwirkung auf einen Strahl optimiert wird, welcher seine erste Reflexion am gegenüberliegenden Ring und seine zweite Reflexion außerhalb der Antenne hat.

13. Ein Drehübertrager gemäß irgendeinem der Ansprüche 10 bis 12,
wobei die innere Antenne (211) und/oder die äußere Antenne (221) eine einstellbare Richtwirkung haben, wobei Sendeleistung und Empfängerempfindlichkeit zusammen mit der Leitfähigkeit der reflektierenden Oberfläche des inneren und äußeren Rings für eine vorgegebene Anzahl von Reflexionen zwischen den Ringen (210, 220) konfiguriert sind.

14. Ein Drehübertrager gemäß irgendeinem der vorhergehenden Ansprüche, umfassend mindestens eines der folgenden Merkmale:
a. die innere Antenne (211) und die äußere Antenne (221) sind axial zueinander versetzt,
b. der innere Ring (210) hat ein dämpfendes Material, welches axial an der inneren Antenne befestigt ist,
c. der äußere Ring (220) hat ein dämpfendes Material, welches axial an der äußeren Antenne befestigt ist,
d. die innere Antenne (211) ist in den Kanal (200) gerichtet,
e. die äußere Antenne (221) ist in den Kanal (200) gerichtet,
f. die innere Antenne (211) hin zum äußeren Ring (220) gerichtet,
g. die äußere Antenne (221) hin zum inneren Ring (210) gerichtet.

## Revendications

1. Joint rotatif assurant une liaison de données à grande vitesse entre des parties rotatives, comprenant un canal circulaire (200),
le canal circulaire (200) comprend :
- une bague interne (210) et une bague externe (220),
les deux bagues étant centrées autour d'un axe de rotation commun (110) et pouvant tourner l'une par rapport à l'autre, et
la bague externe (220) ayant un diamètre plus grand que la bague interne (210),
- une antenne interne (211) couplée mécaniquement à la bague interne (210), et
- une antenne externe (221) couplée mécaniquement à la bague externe (220),
dans lequel
l'antenne interne (211) et l'antenne externe (221) sont dirigées dans un espace entre la bague interne (210) et la bague externe (220) et configurées pour une connexion de signal micro-ondes (169) entre elles,
**caractérisé en ce que**
la distance entre la bague interne et la bague externe représente 5 fois la longueur d'onde du signal micro-ondes avec une tolérance de plus ou moins 50 %.

2. Joint rotatif selon la revendication 1, dans lequel une première paroi latérale (230) axialement sur un côté des bagues et une deuxième paroi latérale (240) opposée à la première paroi latérale (230) axialement sur l'autre côté des bagues sont prévues.

3. Joint rotatif selon la revendication 2,
dans lequel la bague interne (210) est montée sur la première paroi latérale (230) et la bague externe (220) est montée sur la deuxième paroi latérale (240) et
un premier écartement est formé entre la bague interne (210) et la deuxième paroi latérale (240) et un deuxième écartement est formé entre la bague externe (220) et la première paroi latérale (230).

4. Joint rotatif selon la revendication 2,
dans lequel l'une de la bague interne (210) ou de la bague externe (220) est montée sur la première paroi latérale (230) et la deuxième paroi latérale (240), formant un écartement sur chaque côté de l'autre de la bague externe (220) ou de la bague interne (210) par rapport à la première paroi latérale (230) et à la deuxième paroi latérale (240).

5. Joint rotatif selon la revendication 3 ou 4,
dans lequel au moins un des écartements est ponté par au moins un contact coulissant, un joint statique, un joint d'étanchéité ou un chevauchement assurant un couplage capacitif.

6. Joint rotatif selon l'une quelconque des revendications 2 à 5,
dans lequel la première paroi latérale (230) et/ou la deuxième paroi latérale (240) sont en forme de disque et comportent un trou central.

7. Joint rotatif selon l'une quelconque des revendications 2 à 6,
dans lequel au moins une des bagues (210, 220) est en un matériau électromagnétiquement réfléchissant, par exemple un matériau conducteur, ou comporte une surface conductrice, ou au moins une des bagues (210, 220) et/ou des parois latérales (230, 240) est en un matériau diélectrique à haute permittivité.

8. Joint rotatif selon l'une quelconque des revendications 2 à 7,
dans lequel au moins une de la première paroi latérale (230) et de la deuxième paroi latérale (240) inclut un matériau absorbant et/ou réfléchissant pour le signal micro-ondes ou inclut une combinaison d'un matériau absorbant monté sur une surface conductrice.

9. Joint rotatif selon l'une quelconque des revendications 2 à 8,
dans lequel le canal circulaire (200) définit une largeur (251) entre les parois latérales (230, 240) et une hauteur (252) entre les bagues (210, 220).

10. Joint rotatif selon l'une quelconque des revendications précédentes,
dans lequel le canal circulaire (200) renferme un volume cylindrique creux.

11. Joint rotatif selon la revendication 10,
dans lequel soit l'antenne interne (211) et/ou l'antenne externe (221) ont une directivité réglable soit l'antenne interne (211) et/ou l'antenne externe (221) ont une directivité réglable où au moins l'angle de rayonnement est configuré pour une valeur prédéterminée ou une valeur dépendante de la position.

12. Joint rotatif selon l'une quelconque des revendications 10 ou 11,
dans lequel la directivité d'antenne est optimisée pour un faisceau qui a sa première réflexion sur la bague opposée et sa deuxième réflexion à l'extérieur de l'antenne.

13. Joint rotatif selon l'une quelconque des revendications 10 à 12,
dans lequel l'antenne interne (211) et/ou l'antenne externe (221) ont une directivité réglable où la puissance émise et la sensibilité de récepteur, conjointement avec la conductivité de la surface réfléchissante de la bague interne et de la bague externe sont configurées pour un nombre prédéterminé de réflexions entre les bagues (210, 220).

14. Joint rotatif selon l'une quelconque des revendications précédentes,
comprenant au moins une des particularités suivantes :
a. l'antenne interne (211) et l'antenne externe (221) sont décalées axialement l'une par rapport à l'autre,
b. la bague interne (210) comporte un matériau atténuant fixé axialement à l'antenne interne,
c. la bague externe (220) comporte un matériau atténuant fixé axialement à l'antenne externe,
d. l'antenne interne (211) est dirigée dans le canal (200),
e. l'antenne externe (221) est dirigée dans le canal (200),
f. l'antenne interne (211) est dirigée vers la bague externe (220),
g. l'antenne externe (221) est dirigée vers la bague interne (210).
